(19) Europäisches Patentamt
European Patent Office
Office européen des brevets

(11) **EP 3 513 801 A1**

(12) **EUROPEAN PATENT APPLICATION**

(43) Date of publication:
**24.07.2019 Bulletin 2019/30**

(51) Int Cl.:
***A61K 38/10*** *(2006.01)*

(21) Application number: **18203652.5**

(22) Date of filing: **31.10.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.01.2018 KR 20180007536**
**25.10.2018 KR 20180128427**

(71) Applicant: **Intelligent Synthetic Biology Center Daejeon 34141 (KR)**

(72) Inventors:
• **KIM, Sun-Chang**
  **34141 Daejeon (KR)**
• **HWANG, Young Eun**
  **34141 Daejeon (KR)**
• **BUI, Le Minh**
  **34141 Daejeon (KR)**

(74) Representative: **Gassner, Birgitta et al**
**REDL Life Science Patent Attorneys**
**Donau-City-Straße 11**
**1220 Wien (AT)**

(54) **PRODUCING METHOD OF ANTIMICROBIAL PEPTIDE WITH ENHANCED ADHESION AND USES THEREOF**

(57) The present invention relates to a method for preparing an antimicrobial peptide with enhanced adhesion, and an antimicrobial coating method of the antimicrobial peptide with enhanced adhesion. The antimicrobial coating method of the present invention can be widely used as a method for adding an antimicrobial property to medical products and industrial products, because the method is capable of coating the antimicrobial peptide with enhanced adhesion, which has no cytotoxicity and possesses an excellent antimicrobial activity, on various surfaces with optimal efficiency.

Fig. 2

```
<110>      Intelligent Synthetic Biology Center

<120>      Producing method of antimicrobial peptide with enhanced adhesion
           and uses thereof

<130>      OPA18427

<150>      KR 10-2018-0007536
<151>      2018-01-22

<150>      KR 10-2018-0128427
<151>      2018-10-25

<160>      2

<170>      KoPatentIn 3.0

<210>      1
<211>      19
<212>      PRT
<213>      Artificial Sequence

<220>
<223>      NKC sequence


<400>      1
Ala Pro Lys Ala Met Lys Leu Leu Lys Lys Leu Leu Lys Leu Gln Lys
  1               5                  10                  15

Lys Gly Ile



<210>      2
<211>      5
<212>      PRT
<213>      Artificial Sequence

<220>
<223>      linker sequence


<400>      2
Gly Gly Gly Gly Ser
  1               5
```

SEQUENCE LISTING

<110>  Intelligent Synthetic Biology Center

<120>  Producing method of antimicrobial peptide with enhanced adhesion
       and uses thereof

<130>  OPA18427

<150>  KR 10-2018-0007536
<151>  2018-01-22

<150>  KR 10-2018-0128427
<151>  2018-10-25

<160>  6

<170>  PatentIn version 3.5

<210>  1
<211>  19
<212>  PRT
<213>  Artificial Sequence

<220>
<223>  NKC sequence

<400>  1

Ala Pro Lys Ala Met Lys Leu Leu Lys Lys Leu Leu Lys Leu Gln Lys
1               5                   10                  15


Lys Gly Ile



<210>  2
<211>  5
<212>  PRT
<213>  Artificial Sequence

<220>
<223>  linker sequence

<400>  2

Gly Gly Gly Gly Ser
1               5


<210>  3
<211>  30
<212>  PRT
<213>  Artificial Sequence

<220>
<223>  DOPA sequence


<220>
<221>  MISC_FEATURE
<222>  (30)..(30)

<223> X is DOPA

<400> 3

Ala Pro Lys Ala Met Lys Leu Leu Lys Lys Leu Leu Lys Leu Gln Lys
1               5                   10                  15

Lys Gly Ile Gly Gly Gly Gly Ser Gly Gly Gly Gly Ser Xaa
            20                  25                  30

<210> 4
<211> 32
<212> PRT
<213> Artificial Sequence

<220>
<223> DOPA sequence

<220>
<221> MISC_FEATURE
<222> (30)..(32)
<223> X is DOPA

<400> 4

Ala Pro Lys Ala Met Lys Leu Leu Lys Lys Leu Leu Lys Leu Gln Lys
1               5                   10                  15

Lys Gly Ile Gly Gly Gly Gly Ser Gly Gly Gly Gly Ser Xaa Xaa Xaa
            20                  25                  30

<210> 5
<211> 34
<212> PRT
<213> Artificial Sequence

<220>
<223> DOPA sequence

<220>
<221> MISC_FEATURE
<222> (30)..(34)
<223> X is DOPA

<400> 5

Ala Pro Lys Ala Met Lys Leu Leu Lys Lys Leu Leu Lys Leu Gln Lys
1               5                   10                  15

Lys Gly Ile Gly Gly Gly Gly Ser Gly Gly Gly Gly Ser Xaa Xaa Xaa
            20                  25                  30

Xaa Xaa

```
<210>   6
<211>   36
<212>   PRT
<213>   Artificial Sequence

<220>
<223>   DOPA sequence


<220>
<221>   MISC_FEATURE
<222>   (30)..(36)
<223>   X is DOPA

<400>   6

Ala Pro Lys Ala Met Lys Leu Leu Lys Lys Leu Leu Lys Leu Gln Lys
1               5               10                      15


Lys Gly Ile Gly Gly Gly Gly Ser Gly Gly Gly Gly Ser Xaa Xaa Xaa
            20                      25                      30


Xaa Xaa Xaa Xaa
            35
```

**Description**

**Technical Field**

The present invention relates to a method for preparing an antimicrobial peptide with enhanced adhesion and an antimicrobial coating method of the antimicrobial peptide with enhanced adhesion.

**Background Art**

Recently, infectious diseases are continuously increasing worldwide, and as a result, over 60 billion dollars are spent on the treatment of diseases annually. It has been reported that in the United States and Europe, 50,000 die each year, and more than 1,000 people die annually in the Republic of Korea due to infectious diseases. Antibiotics are used to treat these infectious diseases, but antibiotic-resistant superbacteria have appeared due to excessive use of antibiotics, causing an increase in the mortality rate of infectious diseases (an annual average increase of 15%; announced by the Korea Centers for Disease Control and Prevention). In addition, new antibiotics have not been developed since the 1980s. As infectious diseases have become difficult to treat due to antibiotic-resistant bacteria and the absence of new antibiotics, it is much more important to prevent infection before treatment.

Common infections include contact infections, air infections, and droplet infections, among which contact infections are the most frequent. It has been reported that infection in daily life is caused by a large number of bacteria in cell phones, computers, toothbrushes, *etc.*, which are widely used. For example, it has been reported that there are more than 25,000 bacteria per square inch of cell phones, and that these bacteria also include *Escherichia coli, Staphylococcus aureus,* and *Streptococcus,* which are known as antibiotic-resistant bacteria. In addition, in the case of medical infections occurring in hospitals, it has been reported that 2% to 33% infections are caused by surgical instruments, *etc.,* and in 2014, more than 250 billion Korean Won was spent on the treatment of medical infections in Korea. A representative solution for the prevention of contact infections is to use an antimicrobial coating agent, whereby silver and copper currently account for a large proportion of the antimicrobial coating agent. However, there is a problem in that silver and copper have an insufficient antimicrobial activity and cause cytotoxicity due to their nanostructures. Under the circumstances, the need for a novel antimicrobial coating agent has emerged, and one of the alternatives discovered is a coating agent using a microbial peptide.

Antimicrobial peptides (AMPs) are known to be parts of the innate immunity used by most living organisms to protect themselves against external hazards such as infections. These antimicrobial peptides are widely found in living organisms including animals, plants, and insects, as well as some microorganisms. In addition, the presence of antimicrobial peptides was found for the first time in frogs' skin mucus in the 1960s, and currently, thousands of antimicrobial peptides have been identified in various species, and are being synthesized and investigated. So far, in a variety of species including bacteria, invertebrates, vertebrates, and plants, a total of over 5,500 antimicrobial peptides are known to exist, including 3,900 kinds of naturally occurring antimicrobial peptides and 1,600 kinds of synthetic antimicrobial peptides. It has been reported that among these, about 5,300, 1,100, and 1,500, 10, and 100 kinds of peptides associated with antimicrobial, antiviral, antifungal, antiparasitic, and anti-cancer effects, respectively, have been discovered.

It is known that antimicrobial peptides have a wide range of action, showing activities against microorganisms including bacteria, fungi, viruses, *etc.,* as well as against some cancer cells, and that these peptides have an immediate antimicrobial effect due to their unique mechanism of action. In addition to their excellent antibiotic activity, it has been found that antimicrobial peptides play an important role as intermediates in connecting innate immunity and acquired immunity, and further have a role in the functional regulation of the body, such as wound healing, angiogenesis, *etc.* Further, the correlation of antimicrobial peptides with autoimmune diseases and inflammation is also being studied.

Such antimicrobial peptides do not innately have coating ability. Therefore, in order to use the microbial peptides as coating agents, a method is used in which the surface of the product or material to be coated is treated with chemicals, a reactive group is provided, and then the peptides are coated thereto.

However, the conventional antimicrobial coating technique has a problem in that because the method of immobilizing the peptides on a surface by treating them with chemicals through multi-step reactions is used, the effect thereof is not continuous; or alternatively, the immobilization method is complicated and there is a problem of toxicity of the chemicals used. Accordingly, the demand for an environmentally friendly antimicrobial coating technique is increasing (Gallo J et al., Int. J. Mol. Sci. (2014) 15: 13849-13880, Onaizi SA and Leong SSJ, Biotechnol. Adv. (2011)29:67-74).

Meanwhile, mussels, a marine organism, may securely attach themselves to solid surfaces such as rocks in the sea by producing and secreting adhesive proteins, and thus are not affected by the impact of waves or buoyancy of seawater. Mussel adhesive protein is known as strong natural adhesive, and an essential factor for imparting adhesiveness to the mussel adhesive protein is L-3,4-dihydroxyphenylalanine (DOPA) present in proteins. Compared to chemical synthetic adhesives, mussel adhesive protein exhibits about two times higher tensile strength than most epoxy resins, while maintaining flexibility. In addition, mussel adhesive protein may adhere to various surfaces such as plastic, glass, metal, Teflon, biomaterials, and the like, and it may also adhere to wet surfaces within a few minutes, which is a problem that has yet to be fully solved for chemical adhesives.

Accordingly, the present inventors have made intensive efforts to impart an adhesive property to an antimicrobial peptide by an environmentally friendly method, and as a result, they completed the present invention by imparting the adhesive property to an antimicrobial peptide by conjugating DOPA, which is a non-natural amino acid constituting mussel adhesive protein, to a C-terminus of the antimicrobial peptide, and by setting an optimal coating method by confirming the antimicrobial activity according to the number of the conjugated DOPA moieties and coating time.

**Technical Problem**

An object of the present invention is to provide an antimicrobial peptide with enhanced adhesion, wherein a linker and 3,4-dihydroxyphenylalanine (DOPA) are conjugated to an end of an antimicrobial peptide.

Another object of the present invention is to provide a method for preparing an antimicrobial peptide with enhanced adhesion, comprising conjugating a linker and 3,4-dihydroxyphenylalanine (DOPA) to an end of an antimicrobial peptide to prepare an antimicrobial peptide with enhanced adhesion.

Still another object of the present invention is to provide an antimicrobial coating method, comprising:

(a) conjugating a linker and 3,4-dihydroxyphenylalanine (DOPA) to an end of an antimicrobial peptide to prepare an antimicrobial peptide with enhanced adhesion; and
(b) coating the antimicrobial peptide with enhanced adhesion on a surface.

Still another object of the present invention is to provide a composition for antimicrobial coating, comprising the antimicrobial peptide with enhanced adhesion.

**Technical Solution**

In order to solve the above objects, an aspect of the present invention provides an antimicrobial peptide with enhanced adhesion, wherein a linker and 3,4-dihydroxyphenylalanine (DOPA) are conjugated to an end of an antimicrobial peptide.

Hereinbelow, the preparation of the antimicrobial peptide with enhanced adhesion according to the present invention and a use thereof will be described in detail.

As used herein, the term "antimicrobial peptide with enhanced adhesion" refers to a peptide having an antimicrobial activity of an antimicrobial peptide and an adhesive property of DOPA by linking a linker to an end of the antimicrobial peptide and by linking DOPA to an end of the linker.

As used herein, the term "antimicrobial peptide" refers to a part of the innate immune response in a living organism, and thus is referred to as a host defense peptide (HDP). In addition, the antimicrobial peptide also refers to a bio-derived antimicrobial substance composed of amino acids. Since the antimicrobial peptide exhibits an antimicrobial activity by destroying cell membranes or binding to an intracellular substance to kill bacteria, it is unlikely to cause resistance. In addition, since the antimicrobial peptide can kill not only gram-negative and gram-positive bacteria but also viruses or some cancer cells, it has an activity in a wide range of microorganisms. Further, the antimicrobial peptide has an advantage in that it is easily decomposed after the antimicrobial action, and thus is safe for hosts.

The antimicrobial peptide of the present invention may be any peptide as long as it is an antimicrobial peptide that does not lose its antimicrobial activity by conjugating to a linker and DOPA, and specifically the antimicrobial peptide may be NKC.

The NKC is an antimicrobial peptide including the amino acid sequence of SEQ ID NO: 1, has an antimicrobial activity similar to that of a conventional chemical antibiotic, exhibits a cell regeneration effect through an epidermal growth factor receptor (EGFR), and does not show cytotoxicity. Therefore, the NKC is effective for an antimicrobial use *in vivo.*

The NKC may include amino acids added or deleted in the amino acid sequence of SEQ ID NO: 1 as long as its antimicrobial activity is not reduced.

As used herein, the term "linker" refers to a polypeptide that links two proteins, peptides, or amino acids, including the amino acid sequence of SEQ ID NO: 2.

The linker can increase the adhesion of the antimicrobial peptide by minimizing mutual activity inhibition between the antimicrobial peptide and DOPA while linking the antimicrobial peptide and DOPA in the conjugation process.

The types of the linker may include a GS linker (repeats (*e.g.,* 2, 3, 4, *etc.*) of GGGGS, which is comprised of four glycine units and one serine unit) and a PT linker (a linker which is comprised of amino acids proline and threonine), but are not limited thereto.

The linker may include amino acids added or deleted in the amino acid sequence of SEQ ID NO: 2 as long as the activity of a protein, peptide, or amino acid is not further reduced.

In an exemplary embodiment of the present invention, a linker repeating the GGGGS sequence twice was added between the C-terminus of the NKC and the N-terminus of the DOPA in order to minimize mutual activity inhibition between the NKC and DOPA (Table 1).

As used herein, the term "3,4-dihydroxyphenylalanine (DOPA)" refers to a kind of α-amino acid. In addition, the aqueous solution is quite unstable; in particular, the alkaline solution is easily oxidized in air and turns red and then black to produce melanin. In the process of melanin production in plant and animal tissues, tyrosine is oxidized by monophenol monooxygenase to produce DOPA, and then the oxidation proceeds again through dopaquinone.

Additionally, the DOPA plays an important role for providing the adhesiveness in mussel adhesive protein, and has an advantage in that it exhibits an excellent adhesiveness on various surfaces and has the adhesiveness in water.

The antimicrobial peptide with enhanced adhesion may be one in which 1 to 7, specifically 3 to 7, and more specifically 5 to 7 DOPA moieties are linked.

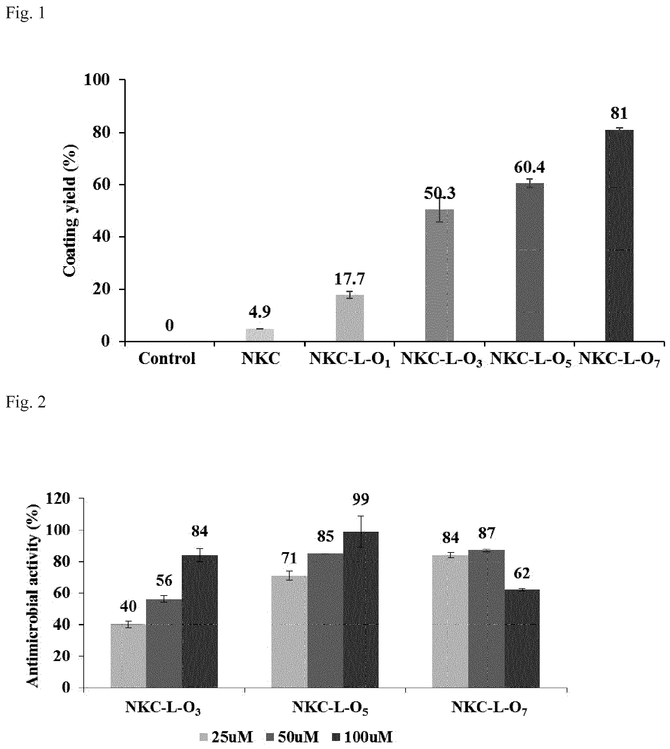

In an exemplary embodiment of the present invention, the coating ability according to the number of the linked DOPA moieties was confirmed, and as a result, it was found that the increase in coating ability was dependent on the number of DOPA moieties. In particular, it was confirmed that when 3 or more DOPA moieties were linked, the coating ability was remarkably increased (Fig. 1).

In another aspect, the present invention provides a method for preparing an antimicrobial peptide with enhanced adhesion, comprising conjugating a linker and 3,4-dihydroxyphenylalanine (DOPA) to an end of an antimicrobial peptide to prepare an antimicrobial peptide with enhanced adhesion.

In the present invention, the terms "antimicrobial peptide with enhanced adhesion" and "antimicrobial peptide" are as described above.

In still another aspect, the present invention provides an antimicrobial coating method, comprising: (a) conjugating a linker and 3,4-dihydroxyphenylalanine (DOPA) to an end of an antimicrobial peptide to prepare an antimicrobial peptide with enhanced adhesion; and (b) coating the antimicrobial peptide with enhanced adhesion on a surface.

In the present invention, the terms "antimicrobial peptide with enhanced adhesion" and "antimicrobial peptide" are as described above.

As used herein, the term "coating" refers to covering the surface of a material with a film. Specifically, the coating of the present invention means that a film consisting of the antimicrobial peptides with enhanced adhesion covers the surface of a specific material so that the surface of the specific material exhibits an antimicrobial activity.

The surface may be polystyrene, polypropylene, polycarbonate, titanium, silicon, *etc.,* and specifically may be polystyrene, titanium, and silicon, but is not limited thereto.

For the coating method in step (b), any method that can be used in the art may be utilized. Non-liming examples thereof include extrusion processing, calendar processing, dipping, spraying, and electrodeposition.

Additionally, in step (b), the coating of the antimicrobial peptide with enhanced adhesion may be carried out under conditions of the peptide concentration of 25 μM to 200 μM, specifically 40 μM to 170 μM, and more specifically 50 μM to 150 μM; and the coating may be carried out for 10 minutes to 12 hours, specifically 2 hours to 11 hours, and more specifically 3 hours to 10 hours, but the conditions are not limited thereto.

In still another aspect, the present invention provides a composition for antimicrobial coating, comprising the antimicrobial peptide with enhanced adhesion.

In the present invention, the terms "antimicrobial peptide with enhanced adhesion", "antimicrobial peptide", and "coating" are as described above.

In an exemplary embodiment of the present invention, the antimicrobial peptide with enhanced adhesion was prepared by conjugating a linker and DOPA to NKC, which is an antimicrobial peptide. In addition, the coating method was set up by analyzing conditions optimized for the coating, such as concentration, number of linked DOPA, and coating time.

Therefore, the present invention implies that an antimicrobial coating composition including the antimicrobial peptide with enhanced adhesion can be provided.

**Advantageous Effects**

The antimicrobial coating method of the present invention is capable of coating the antimicrobial peptide with enhanced adhesion, which has no cytotoxicity and possesses an excellent antimicrobial activity, on various surfaces with optimal efficiency, and thus can be widely used as a method of imparting an antimicrobial property to medical products and industrial products.

**Brief Description of the Drawings**

Fig. 1 is a graph showing the analysis results of adhesion according to the number of DOPA moieties conjugated to NKC.

Fig. 2 is a graph showing the analysis results of an antimicrobial activity according to the number of DOPA moieties conjugated to NKC and the concentration of the adhesive antimicrobial peptide.

Fig. 3 is diagrams showing the analysis results of coating ability according to coating time.

Fig. 4 is graphs showing the analysis results of an antimicrobial activity according to coating time.

Fig. 5 is diagrams showing the analysis results of coating ability according to substrates (polystyrene, titanium, PDMS). Specifically, Fig. 5a is a diagram showing the analysis results of coating ability according to XPS analysis, and Fig. 5b is a diagram showing the analysis results of AFM images.

Fig. 6 is graphs showing the analysis results of an antimicrobial activity according to substrates (polystyrene, titanium, PDMS).

Fig. 7 is a diagram showing an effect of preventing microbial adhesion by the coating of the antimicrobial peptide.

Fig. 8 is a graph identifying the persistence of an antimicrobial activity of the coated antimicrobial peptide.

Fig. 9 is graphs showing the results of the cytotoxicity of the antimicrobial peptide coating at a concentration of 100 $\mu$M.

Fig 10 is graphs showing the results of the cytotoxicity of the antimicrobial peptide coating at a concentration of 200 $\mu$M. Specifically, Fig. 10a is a graph identifying the hemolytic activity of the antimicrobial peptide coating, and Fig. 10b is a graph identifying the cytotoxicity of the antimicrobial peptide coating against human cells.

## Detailed Description of the Embodiment

Hereinbelow, the present invention will be described in detail with accompanying exemplary embodiments. However, the exemplary embodiments disclosed herein are only for illustrative purposes and should not be construed as limiting the scope of the present invention.

## Experimental Example 1: Manufacture of adhesive antimicrobial peptides

## Experimental Example 1-1: Imparting of adhesion to antimicrobial peptides

In order to utilize NKC, which is a peptide exhibiting a strong antimicrobial activity against a wide range of microorganisms, as an antimicrobial coating agent, an adhesive property was imparted to antimicrobial peptides by conjugating 3,4-dihydroxyphenylalanine (DOPA), which is a non-natural amino acid known to play an important role in providing adhesion strength in mussel adhesive proteins, which are biocompatible and exhibit strong adhesion regardless of surfaces, to an end of the NKC. Herein, in order to determine the optimal number of DOPA, 1, 3, 5, or 7 DOPA moieties were conjugated to an end of the NKC, and then GGGGS, which is a flexible linking sequence, was added between NKC and the DOPA sequences to prevent the NKC and DOPA from affecting each other's activity. The amino acid sequence information of the conjugated NKC and DOPA is shown in Table 1 below.

Table 1

| Name | Amino acid sequence* | M. W. | MIC [$\mu$M] |
|---|---|---|---|
| NKC | APKAMKLLKKLLKLQKKGI | 2148.8 | 1 |
| NKC- $(G_4S)_2$- $DOPA_1$ | APKAMKLLKKLLKLQKKGIGGGGGSGGGGSO | 2960.5 | 8 |
| NKC- $(G_4S)_2$- $DOPA_3$ | APKAMKLLKKLLKLQKKGIGGGGGSGGGGSOOO | 3318.2 | 8 |
| NKC- $(G_4S)_2$- $DOPA_5$ | APKAMKLLKKLLKLQKKGIGGGGGSGGGGSOOOOO | 3676.3 | 8 |
| NKC- $(G_4S)_2$- $DOPA_7$ | APKAMKLLKKLLKLQKKGIGGGGGSGGGGSOOOOOOO | 4035.5 | 16 |

*DOPA is abbreviated as "O".

## Experimental Example 1-2: Identification of antimicrobial activity of adhesive antimicrobial peptides

In order to confirm the antimicrobial activity of the adhesive antimicrobial peptides prepared in Experimental Example 1-1, the minimal inhibitory concentration (MIC) which inhibits the growth of *E. coli* was identified by using a method disclosed in Nature Protocols. 2008;3(2):163-175. Briefly, a culture medium in which a single colony had been grown for 16 hours was subcultured and grown until $OD_{600}$ reached 0.4 to 0.6, which corresponds to logarithmic growth. Thereafter, the number of cells was calculated according to the OD values, and the number of cells was set at $10^6$ cell/mL. When a Mueller-Hinton Broth (MHB) was loaded to a polypropylene 96-well plate, 90 $\mu$L was added to the first row of the plate and 100 $\mu$L was added to the control wells, and 50 $\mu$L was added to the remaining wells. Thereafter, the peptide solution was prepared at a concentration of 640 $\mu$M, and 10 $\mu$L thereof was added to each well of the first row. The peptide solution was mixed with the medium using a multi-pipette, and then 50 $\mu$L was transferred to the second row (B). At this time, the peptide was diluted to half concentration, and the peptide was transferred up to the seventh row by the same method so that the peptide (64 $\mu$M to 1 $\mu$M) was dissolved in the medium. $10^6$ cell/mL of *Escherichia coli,* which had been prepared previously, was added thereto, and then the plate was cultured for 18 hours. Thus, the final concentration range of the peptide used in the experiment became 32 $\mu$M to 0.5 $\mu$M. After culturing for 18 hours, the OD values were measured using a microplate reader (Tecan i-control 200 pro), and the MIC values were identified.

As a result, as shown in Table 1, it was found that the MIC value of the NKC to which DOPA was not conjugated was 1, and that the MIC value of the NKC to which a linking sequence and one or more DOPA moieties were conjugated was 8 to 16. Therefore, it was confirmed that the antimicrobial activity of the NKC with enhanced adhesion was reduced. However, the MIC values of this result were considered to be sufficient to kill bacteria, as the values were of similar levels when compared to the MIC values of another antimicrobial peptide (LL-37 (MIC: 4 $\mu$M to 32 $\mu$M)).

**Experimental Example 2: Identification of functionality of coating composition using adhesive antimicrobial peptides**

**Experimental Example 2-1: Identification of coating ability of adhesive antimicrobial peptides according to number of conjugated DOPA** moieties

The level of adhesion according to the number of conjugated DOPA moieties is unknown. Therefore, in order to determine the optimal number of DOPA moieties, the peptides (100 $\mu$M) prepared in Example 1-1 were coated on a polystyrene surface for 12 hours, and then the amount of the attached peptides was identified. Specifically, the peptide solution (100 $\mu$M) was added to a polystyrene 24-well plate in an amount of 300 $\mu$L, and the mixture was coated in an incubator at 37°C for 12 hours. After the coating, the solution in the wells was pipetted to determine its volume, and the concentration of the uncoated peptides was determined by a BCA assay. Finally, the amount of the peptides was calculated considering the volume of the solution after the coating. The calculated amount was compared with the amount of the peptides in the solution before the coating, and then the amount of the peptides present in the solution and on the surface was determined.

As a result, as shown in Table 1, it was confirmed that the coating ability increased as the number of DOPA moieties increased. Particularly, the coating ability increased greatly when the number of DOPA moieties was 3 or more.

**Experimental Example 2-2: Identification of antimicrobial activity in state where adhesive antimicrobial peptides are coated**

In order to confirm the antimicrobial activity in the state where the antimicrobial peptides are coated, the experiment was carried out by an ISO 22196 method in which some steps were changed. Based on the results of Experimental Example 2-1 above, the experiment on the antimicrobial activity was carried out by only using the NKCs to which 3, 5, and 7 DOPA moieties were conjugated (*i.e.*, NKC-L-O$_3$, NKC-L-O$_5$, NKC-L-O$_7$, respectively). Each of NKC-L-O$_3$, NKC-L-O$_5$, and NKC-L-O$_7$ was coated on a polystyrene surface at different concentrations of 25 $\mu$M, 50 $\mu$M, and 100 $\mu$M, and then *E. coli* in logarithmic phase ($10^6$ cell/mL) was cultured on the coated surface for 24 hours. After the culture, the culture medium was diluted to a certain concentration and smeared on a plate, and the CFU values were confirmed after 16 hours. The *E. coli* death level, calculated by comparing the above CFU values with CFU values of the uncoated surface, is represented using the following equation.

$$\text{Antimicrobial Activity} = (1 - \text{CFU of Surface of Sample} / \text{CFU of Control}) \times 100$$

As a result, as shown in Fig. 2, the highest antimicrobial activity was exhibited when coating with NKC-L-O$_5$ (100 $\mu$M), and NKC-L-O$_3$ or NKC-L-O$_5$ showed the antimicrobial activity dependent on the number of DOPA moieties and the concentration of the NKC. It is considered that such results were obtained because the NKC exhibited a concentration-dependent antimicrobial activity and the coating ability was increased according to the number of DOPA moieties.

**Experimental Example 3: Optimization of coating time for adhesive antimicrobial peptide**

**Experimental Example 3-1: Confirmation of coating ability according to coating time**

In order to optimize the coating conditions of NKC-L-$O_5$ (100 $\mu$M), which had been confirmed in Experimental Example 2-2 to have the highest antimicrobial activity, coating degree of the antimicrobial peptide according to coating time was confirmed by X-ray photoelectron spectroscopy (XPS) analysis. XPS is a spectroscopic method in which the surface of a sample is irradiated with X-rays to detect emitted photoelectrons, and the elemental composition and chemical bonding state of the sample surface can be determined based on the binding energy of the detected photoelectrons. When the peptide is coated on the surface, the content of the nitrogen which is not present on the uncoated surface is increased. Based on this, it can be determined that the peptide is present on the surface.

NKC-L-$O_5$ (100 $\mu$M) was coated on the surfaces of polystyrene and titanium for 0 (untreated group), 1, 3, 6, and 12 hours, and then a survey spectrum and a high resolution spectrum on carbon, nitrogen, oxygen, and titanium were identified using Monochromatic Al K$\alpha$ X-ray.

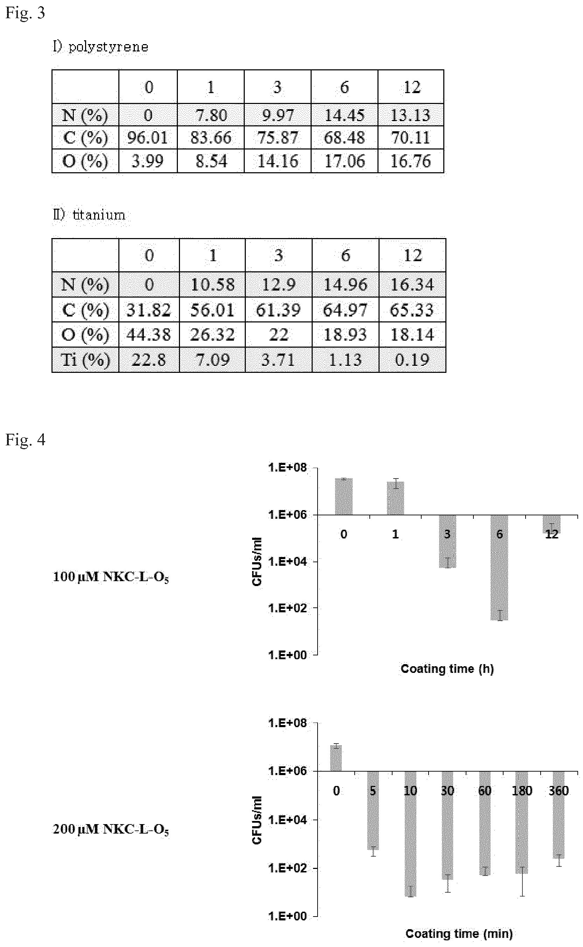

As a result, as shown in Fig. 3, it was confirmed that for both polystyrene and titanium, the content of nitrogen was increased according to the coating time, and for titanium, the content of titanium was decreased. Therefore, it was confirmed that NKC-L-$O_5$ was effectively coated according to the coating time.

**Experimental Example 3-2: Confirmation of antimicrobial activity according to coating time**

In order to measure the antimicrobial activity of NKC-L-$O_5$ (100 $\mu$M) according to coating time, a colony counting assay was carried out. NKC-L-$O_5$ (100 $\mu$M) was coated on a polystyrene 24-well plate for 0 (untreated group), 1, 3, 6, and 12 hours, and then *E. coli* in algebraic logarithmic phase ($10^6$ cell/mL) was cultured on the coated surface for 24 hours. After the culture, an SCDLP medium (300 $\mu$L) was added thereto, and then *E. coli* cells were recovered. Thereafter, these were diluted to a certain concentration and smeared on an LB agar plate. The plate was cultured at 37°C for 16 hours, and then the colonies were counted.

As a result, as shown in Fig. 4, it was confirmed that NKC-L-$O_5$ (100 $\mu$M) showed a coating time-dependent antimicrobial activity up to 6 hours, and that the number of *E. coli* was reduced when coating for 12 hours. Therefore, it can be considered that when NKC-L-$O_5$ at a concentration of 100 $\mu$M is coated for 6 hours, the most excellent antimicrobial activity is exhibited.

Additionally, in order to confirm whether the coating time could be shortened when the concentration of the peptide was doubled, NKC-L-$O_5$ at a concentration of 200 $\mu$M was coated for 5 minutes, 10 minutes, 30 minutes, 1 hour, 3 hours, and 6 hours, and the antimicrobial activity and coating ability thereof were identified using the method specified above. As a result, when coating with NKC-L-$O_5$ (200 $\mu$M) for 10 minutes, the antibacterial activity of NKC-L-$O_5$ (200 $\mu$M) was as strong as when coating with NKC-L-$O_5$ (100 $\mu$M) for 6 hours (>99.999%) (Fig. 4).

**Experimental Example 4: Characteristic analysis of coated surfaces**

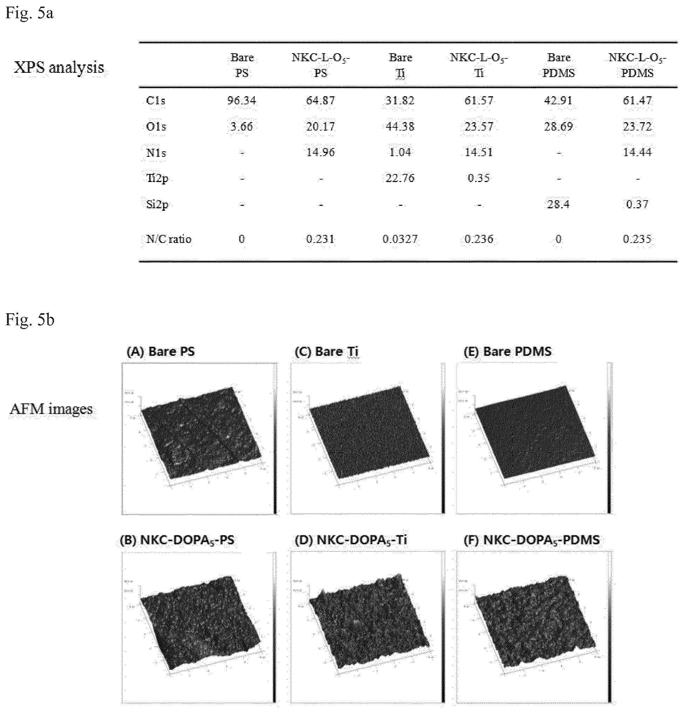

As in Experimental Example 3-1, characteristics of the surfaces of the substrates which had been coated with NKC-L-$O_5$ (200 $\mu$M) for 10 minutes were analyzed by XPS analysis. As a result of testing three substrates (polystyrene, titanium, and PDMS), it was found that the nitrogen (N) content of the NKC-L-$O_5$-coated polystyrene, titanium, and PDMS surfaces was increased by 14% compared to that of the uncoated surfaces, and that in the case of titanium and PDMS surfaces, the Ti content or Si content was greatly decreased after the coating (Fig. 5a). These results suggest that NKC-L-$O_5$ is effectively coated on these three surfaces.

Additionally, the uncoated surfaces and coated surfaces were observed by atomic force microscopy (AFM). AFM is a technique wherein the morphology of surfaces are imaged while the probe touches the surfaces of samples. The surfaces of the three substrates (polystyrene, titanium, and PDMS) after and before the coating were observed by AFM. As a result, it was found that NKC-L-$O_5$ was coated uniformly on each surface (Fig. 5b).

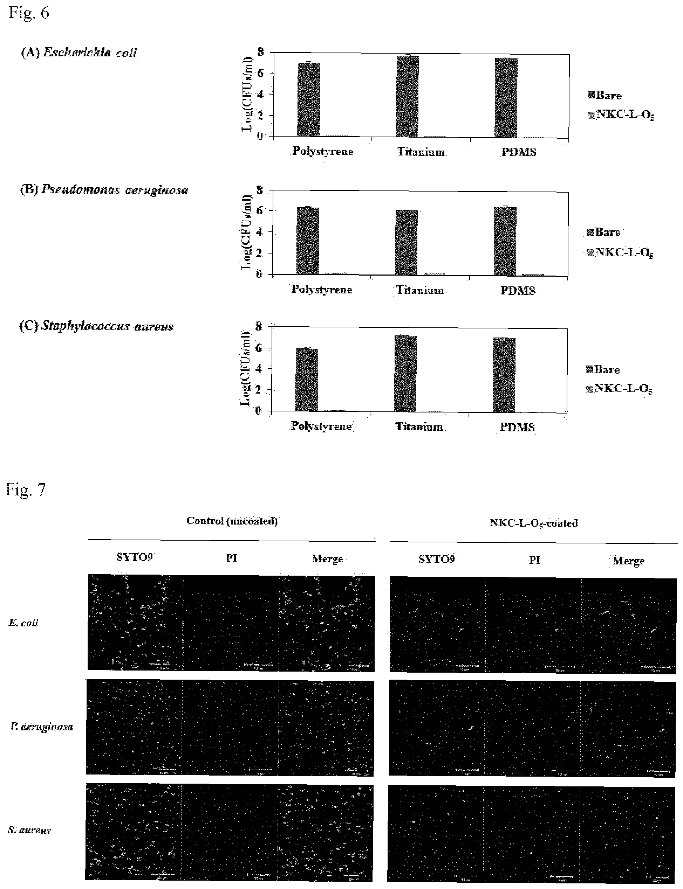

For the coated surfaces, the experiments were carried out as described in Experimental Example 3-2 to determine whether the antimicrobial activity was exhibited against *Escherichia coli, Pseudomonas aeruginosa,* and *Staphylococcus aureus,* which are major infectious strains. As a result, when each of these three strains was cultured on the coated surfaces for 2 hours, all of them were killed (Fig. 6).

A trace amount of bacteria remaining on the surfaces may form a biofilm. Therefore, in order to confirm whether, when coating the surfaces with NKC-L-$O_5$, the bacteria cannot bind to and grow on the surfaces, or whether the cells remaining on the surfaces are live cells or dead cells, *Escherichia coli, Pseudomonas aeruginosa,* and *Staphylococcus aureus* at a concentration of $10^6$ were cultured on the uncoated surfaces and the peptide-coated confocal chamber slide surfaces at 37°C for 24 hours. Thereafter, the surfaces were washed with a 0.85% NaCl buffer, and the bacteria binding on the surfaces were stained with SYTO9 and propidium iodide (PI), and then these were observed by a confocal laser scanning microscope. SYTO9 is a green dye that can stain both live bacteria and dead bacteria, and PI is a dye that can only stain dead bacteria. As a result, a large amount of live bacteria was present on the uncoated surfaces, and on the NKC-DOPA5-coated surfaces, a very small amount of dead bacteria was present in a state where the morphology thereof changed (Fig. 7). These results confirmed that bacteria could not grow on the peptide-coated surfaces.

**Experimental Example 5: Confirmation of persistence of antimicrobial activity**

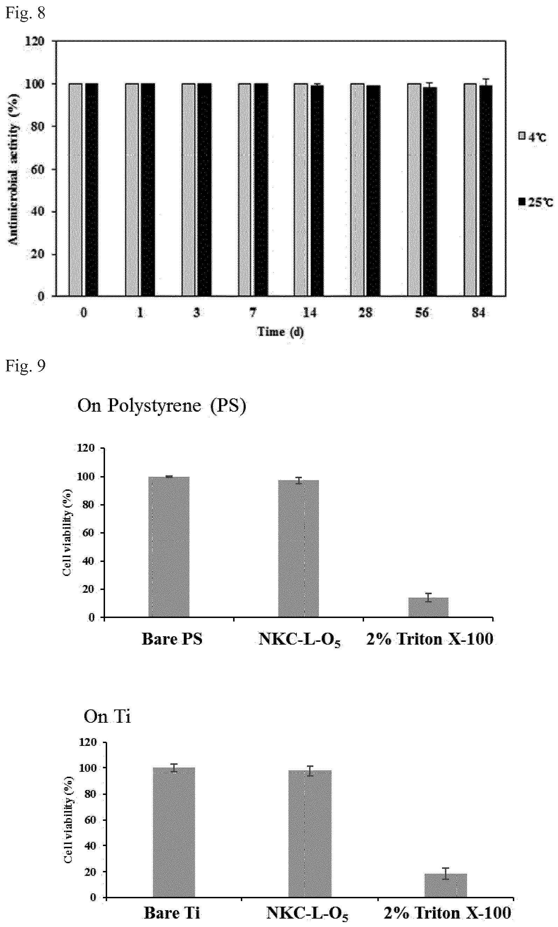

In order to confirm how long the coated peptide can maintain its activity, the 24-well plate in which NKC-L-$O_5$ had been coated was stored at 4°C or 25°C for 1, 3, 7, 14, 28, 56, and 84 days, and the antimicrobial activity was confirmed in the same manner as in Experimental Example 3-2. As a result, when stored at 4°C, a high antimicrobial activity was maintained up to 84 days; and when stored at 25°C, 99.2% of the antimicrobial activity was exhibited (Fig. 8).

**Experimental Example 6: Confirmation of cytotoxicity of adhesive antimicrobial peptide against human cell line**

In order to confirm the cytotoxicity against human cell lines, HaCaT cell lines (*i.e.,* human keratinocytes) were cultured on the polystyrene, titanium, and silicon catheter surfaces coated with NKC-L-$O_5$, and then cell viability was confirmed by an MTT assay. Specifically, HaCaT cells cultured in DMEM (10% FBS, 1% Pen/strep, 1% NEAA) were harvested, and the cells ($5 \times 10^4$ cells per well) were added to a 12-well cell culture plate. The plate was incubated in a $CO_2$ incubator (37°C/5%) for 24 hours. After 24 hours, the culture medium was exchanged with serum-free DMEM, and then an uncoated surface having a size of 1 $cm^2$ or a surface coated with NKC-L-$O_5$ (100 µM) for 6 hours was added to each well. As the negative control group, DMEM containing 2% of Triton X-100, which is a surfactant, was added to culture the cells. After culturing for 24 hours, the surface (1 cm2) and medium were removed, and a DMEM medium containing MTT was added and then cultured in a $CO_2$ incubator (37°C/5%) for 4 hours. After the culture, the solubilization/stop mix solution was added, incubated overnight, and then the absorbance at 570 nm was measured.

As a result, as shown in Fig. 9, it was found that the cell viability of the experimental group, in which the cells were cultured on the NKC-L-$O_5$-coated surface, was not significantly different from that of the control group, in which the cells were cultured on the uncoated surface. Therefore, it was confirmed that NKC-L-$O_5$ is a highly safe peptide which does not affect the growth of human cell lines.

In summary, it was confirmed that the adhesive antimicrobial peptide of the present invention exhibited the most excellent antimicrobial activity when coating in the form of NKC-L-$O_5$ at a concentration of 100 µM for 6 hours, and that the adhesive antimicrobial peptide of the present invention is a highly safe antimicrobial peptide as it does not exhibit cytotoxicity under the above conditions.

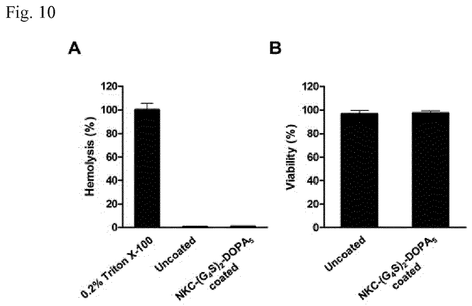

Additionally, it was confirmed that when coating with NKC-L-$O_5$ at a concentration of 200 µM (*i.e.*, the concentration increased by 2-fold), the NKC-L-$O_5$ exhibited a strong antimicrobial activity because it was effectively coated on the surfaces within 10 minutes (Fig. 4), and that the NKC-L-$O_5$ did not show the cytotoxicity against human cells (Fig. 10). In order to verify the hemolytic activity of the adhesive antimicrobial peptide coating, sheep blood was washed with PBS, diluted to 5% (v/v) using the same buffer. Thereafter, 100 µL of the thus-prepared erythrocyte solution was placed in a 96-well plate coated with the adhesive antimicrobial peptide, and allowed to react at 37°C for 1 hour, and then the supernatant obtained by centrifuging the reaction solution was transferred to a new 96-well plate. Thereafter, the absorbance thereof at 415 nm was measured to determine whether hemoglobin leaked due to the damage on an erythrocyte membrane. Herein, the absorbance of a sample treated in the well in which the adhesive antimicrobial peptide had not been coated was defined as 0% hemolysis, and the absorbance of a sample treated with 0.2% triton X-100 was defined as 100% hemolysis. As a result, it was confirmed that the adhesive antimicrobial peptide coating did not exhibit a hemolytic activity (Fig. 10a).

Additionally, in order to confirm the cytotoxicity against human cells when coating with NKC-L-$O_5$ at a concentration of 200 µM, HaCaT cell lines (*i.e.*, human keratinocytes) were placed in a 24-well plate at a concentration of 80,000 cells/well using DMEM containing 10% FBS, and then cultured in the presence of 5% $CO_2$ for 16 hours. PDMS fragments (1 cm2) coated with the adhesive antimicrobial peptide were placed in the wells so that the coated side can be in contact with the cells, and allowed to react at 37°C for 24 hours. Thereafter, the PDMS fragments were removed, and the cell viability was measured using a CellTiter 96-cell proliferation assay kit (Promega Corp.), and then the absorbance at 595 nm was measured. As a result, it was confirmed that the biomaterial (PDMS) coated with the adhesive antimicrobial peptide did not exhibit the cytotoxicity against human cells (Fig. 10b).

In summary, it was confirmed that the adhesive antimicrobial peptide of the present invention also exhibited an excellent antimicrobial activity when coating in the form of NKC-L-$O_5$ at concentrations of both 100 µM and 200 µM, and that the adhesive antimicrobial peptide of the present invention is a highly safe antimicrobial peptide as it does not exhibit cytotoxicity under the above conditions.

From the foregoing, one of ordinary skill in the art to which the present invention pertains will be able to understand that the present invention may be embodied in other specific forms without modifying the technical concepts or essential characteristics of the present invention. In this regard, the exemplary embodiments disclosed herein are only for illustrative purposes and should not be construed as limiting the scope of the present invention. On the contrary, the present invention is intended to cover not only the exemplary embodiments but also various alternatives, modifications, equivalents, and other embodiments that may be included within the spirit and scope of the present invention as defined by the appended claims.

## Claims

1. An antimicrobial peptide with enhanced adhesion, wherein a linker and 3,4-dihydroxyphenylalanine (DOPA) are conjugated to an end of an antimicrobial peptide.

2. The antimicrobial peptide of claim 1, wherein the antimicrobial peptide is NKC comprising an amino acid sequence of SEQ ID NO: 1.

3. The antimicrobial peptide of claim 1, wherein the linker comprises an amino acid sequence of SEQ ID NO: 2.

4. The antimicrobial peptide of claim 1, wherein the linker minimizes mutual activity inhibition between the antimicrobial peptide and the DOPA.

5. The antimicrobial peptide of claim 1, wherein the DOPA is linked as a group of 3 to 7.

6. A method for preparing an antimicrobial peptide with enhanced adhesion, comprising conjugating a linker and 3,4-dihydroxyphenylalanine (DOPA) to an end of an antimicrobial peptide to prepare an antimicrobial peptide with enhanced adhesion.

7. An antimicrobial coating method, comprising:

   (a) conjugating a linker and 3,4-dihydroxyphenylalanine (DOPA) to an end of an antimicrobial peptide to prepare an antimicrobial peptide with enhanced adhesion; and
   (b) coating the antimicrobial peptide with enhanced adhesion on a surface.

8. The antimicrobial coating method of claim 7, wherein the coating is carried out by coating the antimicrobial peptide with enhanced adhesion at a concentration of 50 $\mu$M to 200 $\mu$M.

9. The antimicrobial coating method of claim 7, wherein the coating is carried out for 10 minutes to 10 hours.

10. A composition for antimicrobial coating, comprising the antimicrobial peptide with enhanced adhesion of any one of claims 1 to 5.

Fig. 1

Fig. 2

Fig. 3

I) polystyrene

|        | 0     | 1     | 3     | 6     | 12    |
|--------|-------|-------|-------|-------|-------|
| N (%)  | 0     | 7.80  | 9.97  | 14.45 | 13.13 |
| C (%)  | 96.01 | 83.66 | 75.87 | 68.48 | 70.11 |
| O (%)  | 3.99  | 8.54  | 14.16 | 17.06 | 16.76 |

II) titanium

|        | 0     | 1     | 3     | 6     | 12    |
|--------|-------|-------|-------|-------|-------|
| N (%)  | 0     | 10.58 | 12.9  | 14.96 | 16.34 |
| C (%)  | 31.82 | 56.01 | 61.39 | 64.97 | 65.33 |
| O (%)  | 44.38 | 26.32 | 22    | 18.93 | 18.14 |
| Ti (%) | 22.8  | 7.09  | 3.71  | 1.13  | 0.19  |

Fig. 4

**100 µM NKC-L-O$_5$**

**200 µM NKC-L-O$_5$**

Fig. 5a

| XPS analysis | | Bare PS | NKC-L-O$_5$-PS | Bare Ti | NKC-L-O$_5$-Ti | Bare PDMS | NKC-L-O$_5$-PDMS |
|---|---|---|---|---|---|---|---|
| | C1s | 96.34 | 64.87 | 31.82 | 61.57 | 42.91 | 61.47 |
| | O1s | 3.66 | 20.17 | 44.38 | 23.57 | 28.69 | 23.72 |
| | N1s | - | 14.96 | 1.04 | 14.51 | - | 14.44 |
| | Ti2p | - | - | 22.76 | 0.35 | - | - |
| | Si2p | - | - | - | - | 28.4 | 0.37 |
| | N/C ratio | 0 | 0.231 | 0.0327 | 0.236 | 0 | 0.235 |

Fig. 5b

AFM images

(A) Bare PS  (C) Bare Ti  (E) Bare PDMS

(B) NKC-DOPA$_5$-PS  (D) NKC-DOPA$_5$-Ti  (F) NKC-DOPA$_5$-PDMS

Fig. 6

(A) *Escherichia coli*

(B) *Pseudomonas aeruginosa*

(C) *Staphylococcus aureus*

Fig. 7

Fig. 8

Fig. 9

## On Polystyrene (PS)

## On Ti

Fig. 10

Europäisches
Patentamt

European
Patent Office

Office européen
des brevets

# EUROPEAN SEARCH REPORT

Application Number

EP 18 20 3652

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2011/149173 A1 (KOREA ADVANCED INST SCI & TECH [KR]; KIM SUN CHANG [KR]; JANG SU A [KR] 1 December 2011 (2011-12-01) *cf. abstract, page 1, first para., paras. [10] and [11] at pages 2 and 3, claims 1 and 2* | 1-10 | INV. A61K38/10 |
| Y | DATABASE WPI Week 201753 Thomson Scientific, London, GB; AN 2017-46056Y XP002790146, & KR 2017 0071792 A (KOLLODIS BIOSCIENCES INC) 26 June 2017 (2017-06-26) * abstract * | 1-10 | |
| Y | DATABASE WPI Week 201618 Thomson Scientific, London, GB; AN 2016-01174L XP002790147, & KR 2015 0143173 A (KOLLODIS BIOSCIENCE CO LTD) 23 December 2015 (2015-12-23) * abstract * | 1-10 | |
| X | WO 2009/075788 A1 (SEMPRUS BIOCIENCES CORP [US]; ZHANG ZHENG [US]; O'SHAUGHNESSEY WILLIAM) 18 June 2009 (2009-06-18) *cf. abstract, claims 1, 12,13 and 21* | 1-10 | **TECHNICAL FIELDS SEARCHED (IPC)** A61K |

The present search report has been drawn up for all claims

| Place of search | Date of completion of the search | Examiner |
|---|---|---|
| Munich | 28 March 2019 | Stoltner, Anton |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 18 20 3652

This annex lists the patent family members relating to the patent documents cited in the above-mentioned European search report.
The members are as contained in the European Patent Office EDP file on
The European Patent Office is in no way liable for these particulars which are merely given for the purpose of information.

28-03-2019

| Patent document cited in search report | | Publication date | Patent family member(s) | | Publication date |
|---|---|---|---|---|---|
| WO 2011149173 | A1 | 01-12-2011 | EP | 2575851 A1 | 10-04-2013 |
| | | | JP | 5646051 B2 | 24-12-2014 |
| | | | JP | 2013528617 A | 11-07-2013 |
| | | | KR | 20110130898 A | 06-12-2011 |
| | | | US | 2013244949 A1 | 19-09-2013 |
| | | | WO | 2011149173 A1 | 01-12-2011 |
| KR 20170071792 | A | 26-06-2017 | NONE | | |
| KR 20150143173 | A | 23-12-2015 | NONE | | |
| WO 2009075788 | A1 | 18-06-2009 | US | 2009149673 A1 | 11-06-2009 |
| | | | WO | 2009075788 A1 | 18-06-2009 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GALLO J et al.** *Int. J. Mol. Sci.,* 2014, vol. 15, 13849-13880

- **ONAIZI SA ; LEONG SSJ.** *Biotechnol. Adv.,* 2011, vol. 29, 67-74
- *Nature Protocols,* 2008, vol. 3 (2), 163-175